# EUROPEAN PATENT SPECIFICATION

(11) **EP 4 547 688 B1**
(45) Date of publication and mention of the grant of the patent: **18.02.2026**
(21) Application number: 23736351.0
(22) Date of filing: 30.06.2023
(51) Int. Cl.: C07K 14/005, C12N 15/86, A61K 39/12, A61K 39/29

(54) **VSV VECTOR-ENCODED HCV ENVELOPE PROTEINS E1/E2 AS VACCINES AGAINST HEPATITIS C VIRUS**
VSV-VEKTOR-CODIERTE HCV-HÜLLENPROTEINE E1/E2 ALS IMPFSTOFFE GEGEN HEPATITIS-C-VIRUS
PROTÉINES E1/E2 D'ENVELOPPE DE VHC CODÉES DANS UN VECTEUR DE VSV EN TANT QUE VACCINS CONTRE LE VIRUS DE L'HÉPATITE C

(30) Priority: 01.07.2022 EP 22182552
(43) Date of publication of application: 07.05.2025
(73) Proprietor: Twincore, Zentrum für Experimentelle und Klinische Infektionsforschung GmbH, 30625 Hannover (DE); Universität Bern, 3012 Bern (CH); Institut für Virologie und Immunologie (IVI), 3147 Mittelhäusern (CH)
(72) Inventor: LABUHN, Maurice, 30823 Garbsen (DE); BANKWITZ, Dorothea, 30966 Hemmingen (DE); ZIMMER, Gert, 3006 Bern (CH); PIETSCHMANN, Thomas, 30625 Hannover (DE)
(74) Representative: Moré, Solveig Helga
(86) International application number: PCT/EP2023/067982
(87) International publication number: WO 2024/003343

(56) References cited:
- US-A1- 2003 021 805
- BANKWITZ DOROTHEA ET AL: "Hepatitis C reference viruses highlight potent antibody responses and diverse viral functional interactions with neutralising antibodies", vol. 70, no. 9, 15 December 2020 (2020-12-15), UK, pages 1734 - 1745, XP055859489, ISSN: 0017-5749, Retrieved from the Internet <URL:https://gut.bmj.com/content/gutjnl/70/9/1734.full.pdf?with-ds=yes> DOI: 10.1136/gutjnl-2020-321190

## Description

The present invention relates to the field of vaccination, in particular, of vaccination against hepatitis C virus (HCV). The present invention provides a composition comprising at least parts of the HCV core protein, and HCV E1 and HCV E2 protein of a specific HCV strain, as well as VSV-G protein. The proteins may be assembled in rVSV-HCV pseudovirus particles. This has been identified to induce particularly advantageous broadly neutralizing antibodies. The invention further provides nucleic acids encoding said HCV proteins and VSV proteins but not encoding VSV-G protein. Vaccines comprising the particles, compositons or nucleic acids are disclosed as useful, in particular, for prophylactic vaccination against HCV. Methods of producing the VSV particles or compositons of the invention and the produced particles and compositons are also subject-matter of the invention.

HCV has chronically infected an estimated 71 million people worldwide and is therefore a global health problem. Of those who are infected, 50%-80% progress to chronicity and are at risk to develop liver cirrhosis and hepatocellular carcinoma.

The advent of highly effective direct-acting antivirals (DAAs) has revolutionised patient care. However, providing access to DAAs for all patients worldwide remains a major public health challenge. In addition, in rare cases, therapy fails due to resistance-associated variants and HCV reinfection is possible after treatment-induced cure.

Thus, a prophylactic vaccine is important for control of HCV disease burden. One approach of vaccine development capitalises on the induction of broadly neutralising antibodies (bNAbs) targeting envelope glycoproteins E1 and E2. Multiple studies support an important role of neutralising antibodies for spontaneous HCV clearance (Pestka et al.. Rapid induction of virus-neutralizing antibodies and viral clearance in a single-source outbreak of hepatitis C. Proc Natl Acad Sci U S A 2007;104:6025-30; Logvinoff et al.. Neutralizing antibody response during acute and chronic hepatitis C virus infection. Proc Natl Acad Sci U S A 2004;101:10149-54; Osburn et al.. Spontaneous control of primary hepatitis C virus infection and immunity against persistent reinfection. Gastroenterology 2010;138:315-24; Raghuraman et al.. Spontaneous clearance of chronic hepatitis C virus infection is associated with appearance of neutralizing antibodies and reversal of T-cell exhaustion. J Infect Dis 2012;205:763-71).

Moreover, passive immune-prophylaxis studies in animals confirm the importance of antibodies in protecting from HCV infection (Youn et al.. Sustained E2 antibody response correlates with reduced peak viremia after hepatitis C virus infection in the chimpanzee. Hepatology 2005;42:1429-36; Law et al.. Broadly neutralizing antibodies protect against hepatitis C virus quasispecies challenge. Nat Med 2008;14:25-7; Vanwolleghem et al.. Polyclonal immunoglobulins from a chronic hepatitis C virus patient protect human liver-chimeric mice from infection with a homologous hepatitis C virus strain. Hepatology 2008;47:1846-55).

However, due to the extraordinary diversity and flexibility of HCV as well as different immune evasion mechanisms of HCV, HCV is a difficult target for development of a vaccine. Further, there are limitations in testing vaccine candidates, as there is no robust animal model for direct protection from HCV infection (Bankwitz et al., Entwicklungsansätze für Impfstoffe gegen Hepatitis-C-Virus-Infektionen. Bundesgesundheitsbl 2022, doi.org/10.1007/s00103-021-03477-9)..

HCV is highly variable and viral isolates are classified into eight genotypes (GTs) and multiple subtypes. The genetic diversity even surpasses that of HIV-1 (Smith et al.. Expanded classification of hepatitis C virus into 7 genotypes and 67 subtypes: updated criteria and genotype assignment web resource. Hepatology 2014;59:318-27).

The envelope proteins are targets of neutralising antibodies and represent the most variable proteins of the virus. Hypervariable regions (HVR), e.g., HVR1 in E2 protein, are highly immunogenic and may lead to induction of antibodies that are not protective. HVR1 may even be deleted without the virus loosing infectivity (Bankwitz et al. Hepatitis C virus hypervariable region 1 modulates receptor interactions, conceals the CD81 binding site and protects conserved neutralizing epitopes. J Virol 84:5751-5763; Prentoe et al., Hypervariable region 1 differentially impacts viability of hepatitis C virus strains of genotypes 1 to 6 and impairs virus neutralisation. J Virol 85:2224-2234). For successful vaccine design, it is crucial to elicit cross-protective antibodies against diverse HCV variants.

However, lack of a robust animal model and use of different experimental systems for quantification of antibody responses complicates comparison of vaccine efficacy. Typically, either infection assays based on retroviral HCV pseudoparticles (HCVpp) or cell culture-derived HCV particles (HCVcc) are used to quantify antibody efficacy. In recent years, several panels of HCVpp or HCVcc were developed (Kinchen et al. Defining breadth of hepatitis C virus neutralization. Front Immunol 2018;9:9). In part, these panels include a large number of different E1-E2 proteins (Tarr et al. Hepatitis C patient-derived glycoproteins exhibit marked differences in susceptibility to serum neutralizing antibodies: genetic subtype defines antigenic but not neutralization serotype. J Virol 2011;85:4246-57). However, in some cases, these panels only encompass GT1-derived glycoproteins (Osburn et al.. Clearance of hepatitis C infection is associated with the early appearance of broad neutralizing antibody responses. Hepatology 2014;59:2140-51; Wasilewski et al. Hepatitis C virus resistance to broadly neutralizing antibodies measured using replication-competent virus and pseudo particles. J Gen Virol 2016;97:2883-93). It is unclear if these GT-selective panels adequately report the entire functional diversity of globally sampled HCV, including GT 1 to 7 strains. In other cases, viruses with E1-E2 genes carrying cell culture-adaptive changes, which may influence virus antibody neutralisation, are included (Gottwein et al.. Development and characterization of hepatitis C virus genotype 1-7 cell culture systems: role of CD81 and scavenger receptor class B type I and effect of antiviral drugs.

Hepatology 2009;49:364-77). Moreover, there are well-documented functional differences between cell entry of HCVpp and HCVcc, including dependence on entry factors (Sainz et al.. Identification of the Niemann-Pick C1-like 1 cholesterol absorption receptor as a new hepatitis C virus entry factor. Nat Med 2012;18:281-5) and susceptibility to membrane fusion inhibitors (He et al.. Repurposing of the antihistamine chlorcyclizine and related compounds for treatment of hepatitis C virus infection. Sci Transl Med 2015;7; Perin et al.. Flunarizine prevents hepatitis C virus membrane fusion in a genotype-dependent manner by targeting the potential fusion peptide within E1. Hepatology 2016;63:49-62). Although there are studies attesting a good congruence between HCVpp and HCVcc neutralisation (Kinchen et al., 2008), HCVpp tend to be more neutralisation sensitive than HCVcc (Wasilewski et al., 2016; Urbanowicz et al.. A diverse panel of hepatitis C virus glycoproteins for use in vaccine research reveals extremes of monoclonal antibody neutralization resistance. J Virol 2015;90:3288-301), and the above-mentioned differences may preclude assessment of important determinants and features of the virus-antibody interplay.

Therefore, a reference panel of viruses for rigorous and balanced quantification of breadth and potency of HCV-specific antibodies across the diversity of globally sampled HCV strains was established (Bankwitz et al., Hepatitis C reference viruses highlight potent antibody responses and diverse viral functional interactions with neutralising antibodies. Gut 2021;70(9):1734-1745): For this, the neutralisation potency of polyclonal immunoglobulins from patients infected with HCV genotype (GT) 1-6 was profiled across 13 HCV strains representing five viral GTs. Using metric multidimensional scaling, HCV neutralisation was plotted onto neutralisation maps. K-means clustering was employed to guide virus clustering and selecting representative strains. Viruses mapped to six distinct neutralisation clusters, in part composed of viruses from different GTs. Viruses differed greatly in neutralisation sensitivity, with GT2a.J6 being most resistant and GT5a.SA13 being most sensitive. There was no correlation between viral neutralisation and genetic distance, indicating functional neutralisation clustering differs from sequence-based clustering. Calibrating reference viruses representing these clusters against purified antibodies from 496 patients infected by GT1 to GT6 viruses readily identified individuals with extraordinary potent and broadly neutralising antibodies. It revealed comparable antibody cross-neutralisation and diversity between specimens from diverse viral GTs, confirming well-balanced reporting of HCV cross-neutralisation across highly diverse human samples.

Virus strains from this panel were e.g. used to identify HCV infected subjects with superior antibody responses, and the most effective antibodies were cloned and analysed (Weber et al., Analysis of antibodies from HCV elite neutralizers identifies genetic determinants of broad neutralization. Immunity 55(2):341-345.e7).

Previous approaches to vaccination are e.g., described in Bankwitz et al., 2022. The only candidate vaccines tested in humans were based on viral vectors, ChAd3 and MVA, each encoding the HCV non-structural proteins NS3, NS4A, NS4B, NS5A and inactivated NS5B (NSmut) used in a heterologous prime boost strategy. Although CD4 and CD8 T cell responses were induced, there was no significant difference in incidence of chronic HCV infection (Capone et al., Modulation of the immune response induced by gene electrotransfer of a hepatitis C virus DNA vaccine in nonhuman primates. J Immunol 2006;177:7462-7471; Swadling et al. A human vaccine strategy based on chimpanzee adenoviral and MVA vectors that primes, boosts and sustains functional HCV-specific T cell memory. Sci Transl Med 2014;6:261ra13).

In contrast, other approaches focused on the induction of antibodies, e.g., inactivated HCV particles obtained from cell culture, which can induce protective antibodies (Akazawa et al., Neutralizing antibodies induced by cell culture-derived hepatitis C virus protect against infection in mice. Gastroenterology 2013;145:447-445.e1-4; Gottwein et al. Viral hepatitis: cell-culture derived HCV - a promising vaccine antigen. Nat Rev Gastroenterol Hepatol 2013;10:508-509; Yokokawa et al., Induction of humoral and cellular immunity by immunization with HCV particles vaccine in a nonhuman primate model. Gut 2018;372-379). These inactivated HCV particles are however difficult to concentrate and purify (Wolf et al., Downstream processing of cell-culture-derived virus particles. Expert Rev Vaccines 2011;1451-1475; Nestola et al., Improved virus purification processes for vaccination and gene therapy. Biotechnl Bioeng 2015;112:843-857).

Vesicular stomatitis virus (VSV)-based vaccine vectors were employed as vectors for generation of HCV virus-like particles (VLP), which induced humoral and cellular immune responses (Ezelle et al., Generation of Hepatitis C Virus-Like Particles by Use of a Recombinant Vesicular Stomatitis Virus Vector. J Virol 2002;76:12325-12334). Similar VSV-based vectors have been successfully used for protection against SARS-CoV-2 pathogenesis in mice (Case et al., Replication-Competent Vesicular Stomatitis Virus Vaccine Vector Protects against SARS-CoV-2-Mediated Pathogenesis in Mice. Cell Host & Microbe 2020; 28:465-474) and for an ebola vaccine (VSV-EBOV; Jones et al., Live attenuated recombinant vaccine protects nonhuman primates against Ebola and Marburg viruses. Nature Medicine 2005;11:786-790).

Tests of VLP encoding E1 and E2 produced in the insect cell line Sf9 by means of a baculovirus however did not leed to induction of neutralising antibodies in 3 out of 4 chimpanzees (Elmowalid et al., Immunization with hepatitis C virus-like particles results in control of hepatitis C virus infection in chimpanzees. PNAS 2007;4:8427-8432). Other VLP were targeted to dendritic cells with a lipopeptide (Chua et al., Hepatitis C VLPs delivered to dendritic cells by a TLR2 targeting lipopeptide results in enhanced antibody and cell-mediated responses PLoS One 2012;7:e47492) or a mixture of different HCV genotypes was used as quadrivalent vaccine candidates (Christiansen et al., Immunological responses following administration of a genotype 1a/1b/2/3a quadrivalent HCV VLP vaccine. Sci Rep 2018;8:6483). HCV glycoproteins were also included in chimeric HBV-HCV particles (Beaumont et al., Chimeric hepatitis B virus/hepatitis C virus envelope proteins elicit broadly neutralizing antibodies and constitute a potential bivalent prophylactic vaccine. Hepatology 2013; 57:1303-1313).

Recent approaches are based on recombinantly produced HCV proteins, e.g., HCV-E1E2 (rE1E2), which was successfully tested in chimpanzees and a clinical phase I study (Choo et al. Vaccination of chimpanzees against infection by the hepatitis C virus. PNAS 1994;91:1294-1298; Frey et al., Safety and immunogenicity of HCV E1E2 vaccine adjuvanted with MF59 administered to healthy adults. Vaccine 2010;28:6367-6373; Law et al., PLoS One 2013;8:e59776; Ray et al., Characterization of antibodies induced by vaccination with hepatitis C virus envelope glycoproteins. J Infect Dis 2010;202:862-866), or a secreted form of the E1E2 heterodimer (Wang et al., Induction of broadly neutralizing antibodies using a secreted form of the hepatitis C virus E1E2 heterodimer as a vaccine candidate. PNAS 2022; 119(11):e2112008119). Most approaches however do not take the genetic variability of HCV into account.

In light of this, the inventors addressed the problem of providing a vaccine suitable for use in prophylactic vaccination of humans against HCV overcoming problems arising with prior art vaccine candidates, e.g., a vaccine suitable for protecting against a plurality, preferably, all HCV strains. This problem is solved by the present invention, in particular, the subject matter of the claims.

The invention provides a composition comprising
a) at least the 60 C-terminal amino acids of HCV core protein,
b) HCV E1 protein of a first HCV strain
c) HCV E2 protein of a first HCV strain, and
d) VSV-G protein,
wherein the first HCV strain is selected from GT2a.J6, GT2r.2r and GT5a.SA13.

Said proteins may be associated to form particles, in particular, rVSV-HCV pseudovirus particles. Thus, the invention also provides rVSV-HCV pseudovirus particles comprising
a) at least the 60 C-terminal amino acids of HCV core protein,
b) HCV E1 protein of a first HCV strain
c) HCV E2 protein of a first HCV strain, and
d) VSV-G protein,
wherein the first HCV strain is selected from GT2a.J6, GT2r.2r and GT5a.SA13. Compositons comprising said particles are also subject-matter of the invention.

GT2a.J6, GT2r.2r and GT5a.SA13 are HCV neutralisation clusters or HCV biotypes defined in Bankwitz et al., 2021. As described therein, there was no correlation between neutralisation and genetic distance. It is understood herein that a HCV strain is selected from a neutralisation cluster if it belongs to said neutralisation cluster.

It is preferred throughout the invention that the first HCV strain belongs to cluster GT2a.J6. Said cluster comprises the strain GT2a. As further shown herein, the inventors were able to demonstrate that in the context of the invention, the E1 and E2 protein from GT2a.J6, e.g. genotype GT2a (Genebank accession No. MT955904, cf. https://www.ncbi.nlm.nih.gov/nuccore/MT955904) induce antibodies with particularly good neutralising characteristics and a high cross-reactivity between strains.

E1 and E2 proteins from a HCV strain belonging to clusters GT2r.2r and GT5a.SA13 were also shown to lead to neutralising antibodies with reasonable cross-reactivity. They can thus alternatively - or additionally - be used. As shown in Bankwitz et al., 2021, cluster GT2r.2r comprises the strains GT2r and GT2k (Genebank accession No. MT955910 (https://www.ncbi.nlm.nih.gov/nuccore/MT955910) and MT955913 (https://www.ncbi.nlm.nih.gov/nuccore/MT955913)). Cluster GT5a.SA13 comprises the strain GT5a.

Other HCV strains or genotypes may be characterized as belonging to one of the clusters using the method described in Bankwitz et al., 2021. This includes artificial HCV virus variants, or HCV variants with modified E1 and E2 proteins. In brief, for this, the neutralisation potency of polyclonal immunoglobulins from a plurality of patients infected with HCV genotype (GT) 1-6 (e.g., at least 50 patients) may be profiled across different HCV strains representing different viral GTs. Using metric multidimensional scaling, HCV neutralisation is plotted onto neutralisation maps, and K-means clustering is employed to guide virus clustering.

As this requires a rather comprehensive analysis, it is preferred to characterize an HCV strain or genotype of interest as belonging to one of the defined clusters by a method comprising steps of:
a) integrating the protein sequences of interest into a complete HCV genome;
b) preparing HCVcc;
c) carrying out a neutralisation assay using 6 different monoclonal antibodies, namely, HC33.8, AR3C, AP33, HC84.27, HEPC3, and HEPC74; preferably, at a concentration of 50 µg antibody/mL, and
d) determining a percentage of neutralisation efficiency for each of said antibodies, and
e) comparing the percentage of neutralisation for each of said antibodies with the percentage of neutralisation of each of said antibiodies towards the respective strain as HCVcc,
wherein the HCV strain or genotype of interest is considered to belong to the same neutralisation cluster if the percentage of neutralisation of each of said antibodies towards said strain of interest is +/- 10% of the percentage of neutralisation of each of said antibodies towards the respective strain as HCVcc.

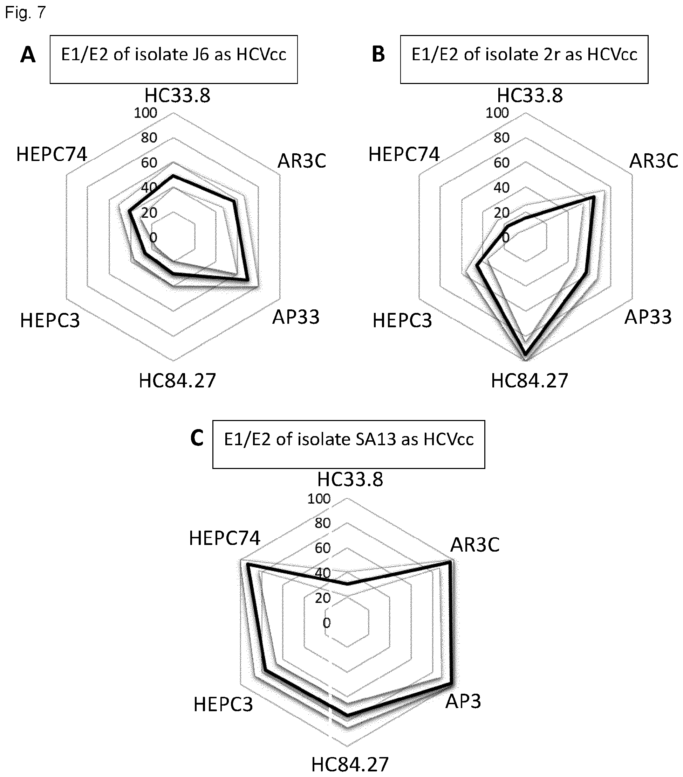

For example, a rVSV-HCV with an E1/E2 protein from a specific strain of interest that has not yet been characterized as belonging to one of the neutralisation clusters of Bankwitz et al., 2021 can be characterized as belonging to the neutralisation cluster of GT2a.J6 if the percentage of neutralisation of each of said antibodies towards said strain of interest is +/- 10% of the percentage of neutralisation of each of said antibodies towards the GT2a.J6 strain as HCVcc. Exemplary preparation of HCVcc is shown in Example 2. A neutralisation fingerprint, i.e., the percentage of neutralisation of each of said antibodies towards the GT2a.J6 strain, the GT2r.2r strain and the GT5a.SA13 strain as HCVcc is provided in Fig. 7. Some or all steps of said method can be carried out as described in the legend, and preferebly, in Example 3.

The sequence identity of the E1 and E2 proteins of said first HCV strain to the E1 and/or E2 proteins of the reference strains for the respective clusters (GT2a, GT2r or GT2k and GT5a), preferably, to both E1 and E2, may be at least 90%, optionally, at least 95%, at least 99% or, preferably, 100%.

For this comparison, the sequence identity in the hypervariable region of the E2 protein (E2 hypervariable region (HVR1) encompassing the first 27 amino acid residues at the N-terminus of E2) is ignored. The sequence identity of different domains or regions of said proteins may vary, in particular, the sequence identity in the hypervariable regions may be as low as 0%, e.g., 10-100%, 20-90%, 30-80%, 40-70% or 50-60%, or said region or regions may be partly or completely missing (e.g., 10%-100%, 20-90%, 30-80%, 40-90% or 50-80% thereof).

Preferably, the HCV E1 protein of said first strain, belonging to the cluster GT2a.J6, has at least 90%, e.g., at least 95% amino acid identity to SEQ ID NO: 1, and/or the HCV E2 protein has at least 90%, e.g., at least 95% amino acid identity to SEQ ID NO: 2 (optionally, excluding aa 1-27, i.e., HVR1). rVSV-HCV pseudovirus particles on this basis are descignated **rVSV-HCV-E1/E2-J6-particles.** In a preferred embodiment, the sequence of the HCV E1 protein comprises or is SEQ ID NO: 1, and/or the sequence of the HCV E2 protein comprises or is SEQ ID NO: 2 (optionally, excluding aa 1-27, i.e., HVR1).

If the HCV of said first strain is from GT2r.2r, the HCV E1 protein has at least 90%, e.g., at least 95% amino acid identity to SEQ ID NO: 4, and/or the HCV E2 protein has at least 90%, e.g., at least 95% amino acid identity to SEQ ID NO: 5 (optionally, excluding aa 1-27, i.e., HVR1). rVSV-HCV pseudovirus particles on this basis are descignated **rVSV-HCV-E1/E2-2r-particles.** Preferably, in that case the sequence of the HCV E1 protein is SEQ ID NO: 4, and/or the sequence of the HCV E2 protein is SEQ ID NO: 5 (optionally, excluding aa 1-27, i.e., HVR1).

If the HCV of said first strain is from GT5a.SA13, the HCV E1 protein has at least 90%, e.g., at least 95% amino acid identity to SEQ ID NO: 7, and/or the HCV E2 protein has at least 90%, e.g., at least 95% amino acid identity to SEQ ID NO: 8 (optionally, excluding aa 1-27, i.e., HVR1). rVSV-HCV pseudovirus particles on this basis are descignated **rVSV-HCV-E1/E2-SA13-particles.** Preferably, in that case, the sequence of the HCV E1 protein is SEQ ID NO: 7, and/or the sequence of the HCV E2 protein is SEQ ID NO: 8 (optionally, excluding aa 1-27, i.e., HVR1).

It has previously been found that the 60 C-terminal amino acids of HCV core protein are required for correct expression and/or processing of E1 and E2 proteins. More amino acids of said core protein may also be contained. Typically, for optimal expression, the HCV core protein also is a HCV core protein of said first HCV strain. However, it is also possible to use HCV core proteins of another HCV strain from the same or another HCV neutralisation cluster. The 60 C-terminal amino acids of HCV core protein may also have at least 90%, e.g., at least 95% sequence identity to a HCV core protein, preferably, from said first strain.

Preferably, only a part of the HCV core protein is used, in particular, a part that does not allow for the generation of HCV virus-like particles together with the E1 and E2 proteins. In Ezelle et al., 2002, a similar approach was used, but they found that the E1/E2 containing particles formed largely did not incorporate VSV-G. This is preferably avoided by using a smaller part of the core protein, e.g., the 60 C-terminal amino acids of HCV core protein.

Preferably, if the HCV strain is from GT2a.J6, the sequence of the 60 C-terminal amino acids of the HCV core protein is SEQ ID NO: 3, the sequence of the HCV E1 protein is SEQ ID NO: 1, and the sequence of the HCV E2 protein is SEQ ID NO: 2 (optionally, excluding aa 1-27, i.e., HVR1).

Optionally, if the HCV strain is from GT2r.2r, the sequence of the 60 C-terminal amino acids of the HCV core protein is SEQ ID NO: 6, the sequence of the HCV E1 protein is SEQ ID NO: 4, and the sequence of the HCV E2 protein is SEQ ID NO: 5 (optionally, excluding aa 1-27, i.e., HVR1). Optionally, if the HCV strain is from GT5a.SA13, the sequence of the 60 C-terminal amino acids of the HCV core protein is SEQ ID NO: 9, the sequence of the HCV E1 protein is SEQ ID NO: 7, and the sequence of the HCV E2 protein is SEQ ID NO: 8 (optionally, excluding aa 1-27, i.e., HVR1).

In addition to the HCV proteins mentioned, VSV-G protein is also comprised in the particles of the invention. This has the advantage that the pseudovirus particles can infect a broader selection of cells. VSV-G allows for clathrin-mediated endocytosis of virus particles into endosomes, and can thus lead to transduction of nearly all cell types. Consequently, a higher *in vivo* reproduction of the virus is reached, and the immune response increased. VSV-G may have the sequence of SEQ ID NO: 16 or at least 80%, e.g., at least 90% or at least 99% sequence identity thereto.

Besides its main function to mediate fusion between the virus envelope and host cellular membrane to a variety of different cells, the VSV-G also stabilises the pseudovirus particles and allows for preparation of a higher titer composition comprising the rVSV-HCV pseudovirus particles.

A pseudovirus particle, also called a pseudovirus, is a virus carrying a foreign viral envelope protein. Thus, in this case, a recombinant VSV virus in addition to VSV-G further comprises the recited HCV proteins.

The particles of the invention preferably do no comprise a nucleic acid encoding VSV-G protein.Thus, the infected cells do no produce further VSV-G protein. Instead, large amounts of E1/E2 protein are formed, and rVSV-HCV pseudovirus particles comprising said HCV proteins, but no VSV-G are generated. The pseudovirus preferably is thus limited replication competent and attenuated compared to wild type VSV. The rVSV-HCV pseudovirus particles are able to accomplish a single-round infection via the highly efficient VSV-G protein that is transcomplemented in the process of the *in vitro* particle production. The emerging progeny pseudovirus particles from infected cells are completely deficient of VSV-G protein. Thereby, the attenuation compared to the wild type VSV encompasses a limited infectious titer and a massively restricted host tropism.

The gene for VSV-G of VSV can be replaced by the sequences encoding the HCV proteins (i.e., at least the 60 C-terminal amino acids of HCV core protein and the E1 and E2 protein).

Without intending to be bound by the theory, it is believed that both the administered rVSV-HCV pseudovirus particles comprising VSV-G administered and the rVSV-HCV pseudovirus particles not comprising VSV-G that are produced by the infected cells contribute to the induction of the desired immune response. In a human subject, said rVSV-HCV pseudovirus particles can further infect liver cells expressing the human receptor proteins occludin and CD81. As further described herein, the inventors found in a mouse experiment with transgenic mice expressing these receptors that this is not detrimental to the immune response, but it also does not appear to be essential.

The particles of the invention may further comprise VSV nucleoprotein, VSV phosphoprotein, VSV matrixprotein, and/or VSV polymerase. These enable the pseudovirus particles of the invention to infect cells productively. Suitably variants may alternatively be used.

The particles of the invention, further may comprise a nucleic acid encoding HCV and, optionally, VSV proteins. Preferably, the nucleic acid encodes said 60 C-terminal amino acids of HCV core protein, HCV E1 protein and HCV E2 protein, VSV nucleoprotein, VSV phosphoprotein, VSV matrixprotein, and VSV polymerase. The coding region preferably is operably linked to a promotor, e.g., the T7 promotor. Preferably, said nucleic acid does not encode VSV-G. The nucleic acid typically is RNA.

The rVSV-HCV particles may further comprise, e.g., at least one further HCV protein selected from the group consisting of P7 (assembly factor), N2, NS3, NS4A, NS4B, NS5A and NS5B, and preferably, the nucleic acid may encode said further HCV protein(s). Alternatively, or additionally, peptides from at least one of said proteins may be comprised in the particles, e.g., comprising at least one, preferably, at least 2, at least 5, at least 10 or at least 20 HCV T cell epitopes, such as T cell epitopes that can be recognized in the context of a plurality of HLA alleles, e.g. HLA alleles that are (together represented in at least 20%, at least 25%, at least 40% or at least 50% of the population and/or that are conserved between different HCV strains. Preferably, a combination of T cell epitopes is selected such that at least 80%, preferably, at least 90% of human individuals can present at least one of said T cell epitope on their HLA. T cell epitopes may be MHCI-restricted of MHCII-restricted T cell epitopes.

Peptides comprising T cell epitopes from different HCV strains and/or from different proteins may be comprised. This may further enhance the immune response and enable generation of additional antibodies to these proteins and/or T cell responses. The amount of additional proteins and/or peptides encoded is limited by the maximal size of the vector. Preferably, the maximal size of the vector is 16-18 kb. In an optimized version, the particles comprise, and the nucleic acid encodes, e.g., full length HCV core protein, E1, E2, P7, and NS2. Other HCV proteins can also be selected, but at least one HCV proteins must be missing, thus avoiding the functional full HCV genome.

The invention also provides a composition comprising rVSV-HCV particles of the invention, and, optionally, a suitable solvent such as a buffer and/or other excipient, e.g., a bulking agent such as trehalose and/or salts (e.g., NaCl, Tris, citrate or phosphate). The composition can be a dry, e.g., a lyophilised composition. Such dry compositions may be advantageous for storage, e.g., at up to 40°C. For administration, the composition typically is a solution comprising a buffer, i.e., a dry composition may be reconstituted. While the composition may comprise an adjuvant, it is one of the advantages of the invention that this is not required. The composition may be a pharmaceutical composition, in particular, a vaccine. It should be suitable for administration to a human, e.g., it should be free of bacteria and other viruses, and non-toxic.
in one embodiment of the invention, the composition comprising rVSV-HCV particles of the invention optionally further comprises rVSV-HCV particles comprising
   a) at least the 60 C-terminal amino acids of HCV core protein,
   b) HCV E1 protein of a second HCV strain and
   c) HCV E2 protein of a second HCV strain,
   d) VSV-G protein,
wherein said second HCV strain is different from said first HCV strain. Optionally, said second HCV strain is a HCV strain selected from GT2a.J6, GT2r.2r and GT5a.SA13. For example, rVSV-HCV-E1/E2-J6-particles may be combined with rVSV-HCV-E1/E2-2r-particles and/or rVSV-HCV-E1/E2-SA13-particles.

In particular, the second HCV strain belongs to a different neutralisation cluster than the first HCV strain. This combination of envelope proteins from different neutralisation clusters may allow for induction of antibodies that react even more broadly against different HCV strains. Of course, the composition may also comprise further rVSV-HCV particles with HCVE1 and E2 proteins of a third or further strain from a still different neutralisation clustur (e.g., to provide a composition comprising rVSV-HCV-E1/E2-J6-particles, rVSV-HCV-E1/E2-2r-particles and rVSV-HCV-E1/E2-SA13-particles), and/or additional antigens or vaccines, such as rVSV-EBOV. This may be useful for combination vaccination.
in one embodiment, the invention also provides a nucleic acid encoding
   a) at least the 60 C-terminal amino acids of HCV core protein,
   b) HCV E1 protein of a first HCV strain,
   c) HCV E2 protein of a first HCV strain, and
   d) VSV nucleoprotein, VSV phosphoprotein, VSV matrixprotein, and VSV polymerase,
wherein the first HCV strain is a HCV strain selected from GT2a.J6, GT2r.2r and GT5a.SA13. Preferably, the coding sequence may be operably linked to a promotor active in human cells, e.g., a T7 promotor. The nucleic acid may also comprise a packaging signal that allows for packaging in the pseudovirus particles of the invention, e.g., the VSV packaging signal.

Said nucleic acid may be DNA, e.g., a plasmid, or RNA. The nucleic acid does not encode VSV-G. It may be used, e.g., to produce rVSV-HCV particles of the invention. The nucleic acid may comprise, e.g., SEQ ID NO: 10 encoding the 60 C-terminal amino acids of HCV core protein, E1 protein and E2 protein of GT2a-J6, SEQ ID NO: 11 encoding the 60 C-terminal amino acids of HCV core protein, E1 protein and E2 protein of GT2r.2r or SEQ ID NO: 12 encoding the 60 C-terminal amino acids of HCV core protein, E1 protein and E2 protein of GT5a.SA13, preferably, SEQ ID NO: 10. It may be RNA, preferably, a recombinant VSV nnucleic acid not encoding VSV-G. Alternatively, it may bei DNA comprising DNA sequences corresponding to said RNA sequences. A preferred rVSV based vector construct encoding the proteins of the VSV particles of the invention, based on GT2a-J6 is provided in SEQ ID NO: 13. An alternative rVSV based vector construct encoding the proteins of the VSV particles of the invention, based on GT2r.2r is provided in SEQ ID NO: 14. An alternative rVSV based vector construct encoding the proteins of the VSV particles of the invention, based on GT5a.SA13 is provided in SEQ ID NO: 15.

Another object of the invention is a method for producing the rVSV-HCV particles of the invention, comprising
a) culturing a cell expressing VSV-G protein and transfected with the nucleic acid of the invention described above under conditions suitable for production of said rVSV-HCV particles, and
b) isolating said particles, and
c) optionally, concentrating said particles, and
d) optionally, formulating said particles in a formulation suitable for storage and/or for administration as a vaccine.

The cell expressing VSV-G protein may be, e.g., a BHK-G43 cell. Expression of VSV-G can be induced by induction with mifepristone. The cells may be transfected with the nucleic acid by transfection with a starter virus comprising said nucleic acid.

Isolation of particles comprises the separation from the particle-producing cells, and, from at least part, preferably, all of the cell culture medium. The particles can be further concentrated. The isolation and/or concentration of the particles may be achieved by ultracentifugation and/or spin column purification systems. E.g. as described above, a formulation suitable for storage may be a dry, e.g., lyophilised, or a dissolved form, e.g., in a buffer. Said formulation for storage is suitable for administration to a human after reconstitution e.g., in water for injection or in a suitable buffer.

rVSV-HCV particles obtainable by said method are also provided by the invention, wherein said particles preferably are the particles of the invention described herein.

The invention also provides a vaccine comprising the rVSV-HCV particles of the invention, the nucleic acid of the invention, or the composition of the invention.

Said vaccine is for use in prophylactic vaccination of a subject. The subject may be a human subject or a mouse subject, preferably, a human subject. Optionally, it is a subject that has successfully been treated for a chronic HCV infection. Unfortunately, therapy of a chronic HCV infection does not protect from reinfections, and the risk is high. In this case, the vaccination of the present invention reduces the risk of reinfection or prevents reinfection. However, the subject may also never have been infected with HCV. The subject may also be another subject at risk for a HCV infection, e.g., a user of injectable drugs, a man having sex with men, a person exposed to an increased risk of nosocomial infection or medical personnel.

The invention also provides a syringe comprising the vaccine of the invention, e.g., in dry from or in solution.

Advantageously, the vaccine of the present invention induces antibodies that can endow a subject with protection from a broad variety of HCV virus strains, e.g., strains belonging to the clusters GT3a.S53, GT2r.2r, GT2b.2b4, GT1b.J4, GT5a.SA13, and GT2a.J6.

The vaccination typically is prophylactic vaccination. Therapeutic vaccination can also be envisioned.

The present invention is further illustrated in the following examples with reference to the accompanying figures and sequences, nevertheless, without being limited thereto.

### Legends

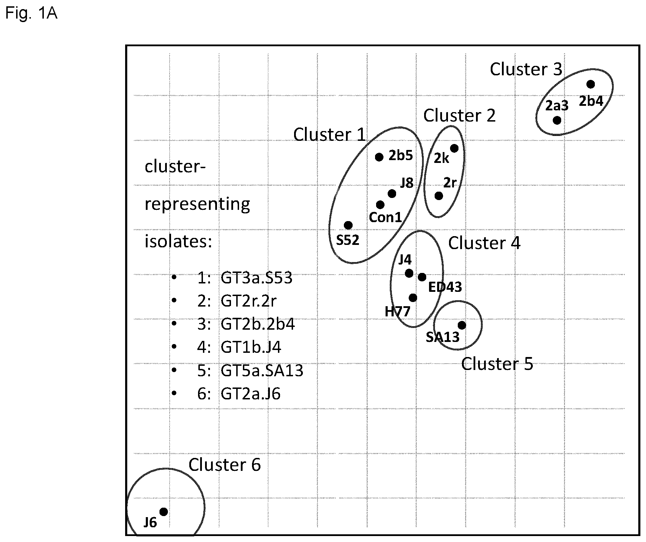

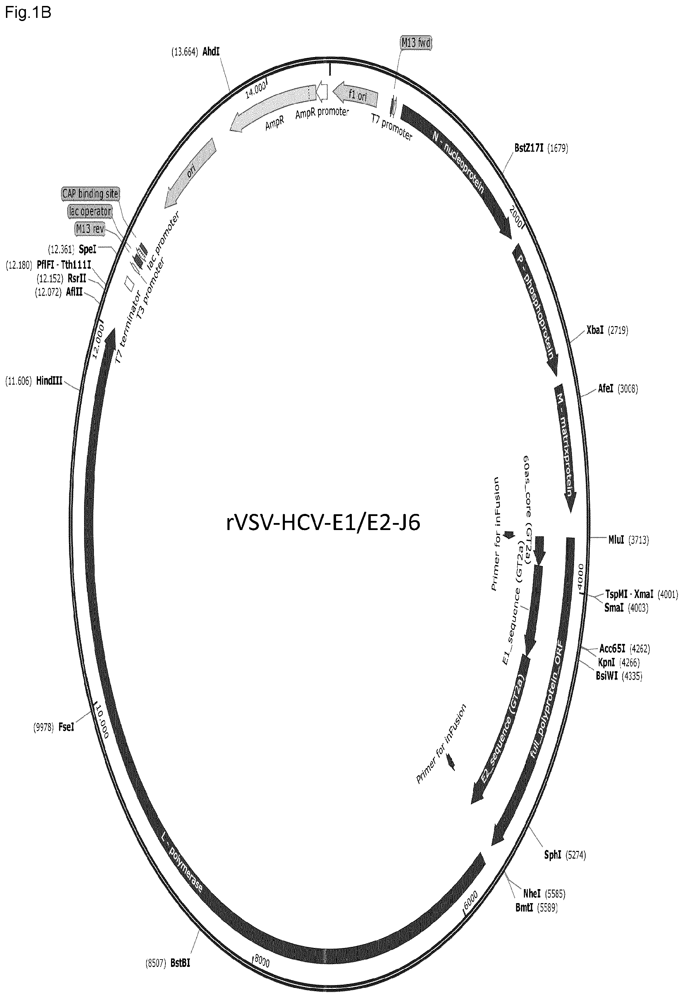

**Fig. 1****: HCV reporter-virus biotype clustering and the rVSV-HCV-E1/E2-J6 construct.** (A) Depiction of 13 HCVcc reference reporter viruses tested for neutralizability by antibodies isolated from patients. A so-called clustering was able to divide the reference viruses into 6 neutralization clusters based on their neutralization phenotype (Bankwitz et al., 2021). Each cluster is represented by a specific biotype (label in figure). The subdivision of these 13 reference viruses or the 6 representative biotypes forms the basis of all further approaches (Figure from: Bankwitz et al., 2021) (B) Exemplary representation of one of the generated rVSV-based vector constructs for production of the rVSV-HCV pseudovirus particles. The glycoprotein (G protein) was deleted from the VSV genome and replaced either by the sequence of GFP (control vector) or by the sequence of the C-terminal 60 amino acids of the HCV core protein plus HCV glycoprotein E1 plus HCV glycoprotein E2. Six constructs were generated: genotype 1b isolate J4 (representing biotype cluster 4), genotype 2a isolate J6 (representing biotype cluster 6), genotype 2b isolate 2b4 (representing biotype cluster 3), genotype 2r isolate 2r (representing biotype cluster 2), genotype 3a isolate S52 (representing biotype cluster 1) and genotype 5a isolate SA13 (representing biotype cluster 5).

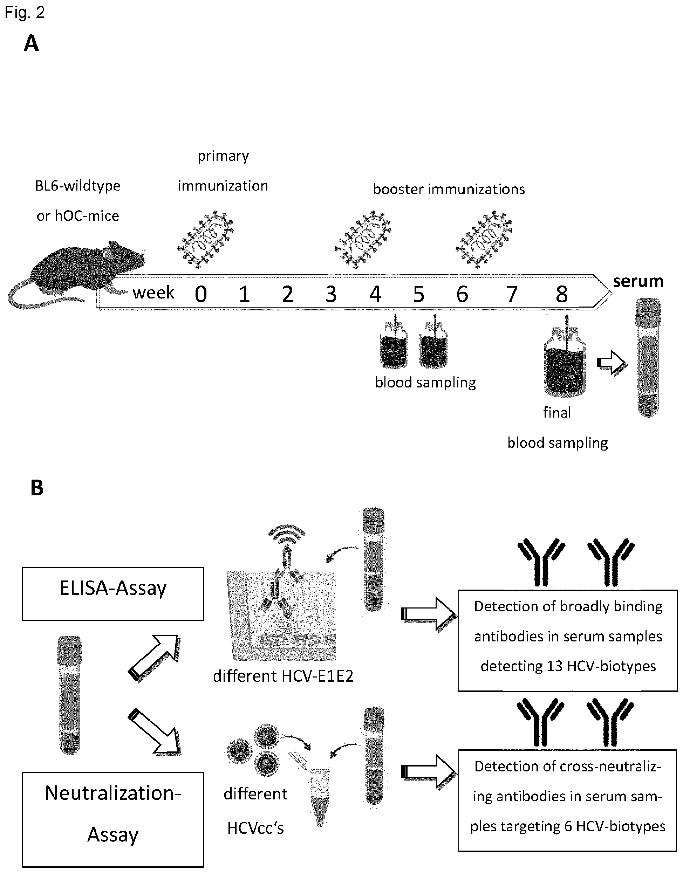

**Fig. 2****: Schematic depictions of the *in vivo* immunization schedule and the *in vitro* follow up test-systems for evaluation of the antibody based reaction in mice.** (A) The *in vivo* immunization studies were performed in C57BL/6 wild-type mice as well as in receptor-transgenic hOC mice (liver-specific expression of the human HCV entry receptors CD81 and occludin). The animals were immunized with 1×10⁸ rVSV-HCV pseudovirus particles per mL injection volume intramuscularly at the beginning of the experiment, after 4 weeks and after 7 weeks. Minimal amounts of blood were drawn at week 5 and week 6 of the experiment, and at week 9 the final maximal blood volume of each animal was drawn. The serum was isolated by centrifugation and used for subsequent assays. (B) The presence of binding antibodies in the serum was tested using an ELISA binding assay against lysates of cells infected with HCVcc 13 reference viruses, the presence of neutralizing antibodies was tested using a neutralization assay against 6 different cell culture-generated reference viruses.

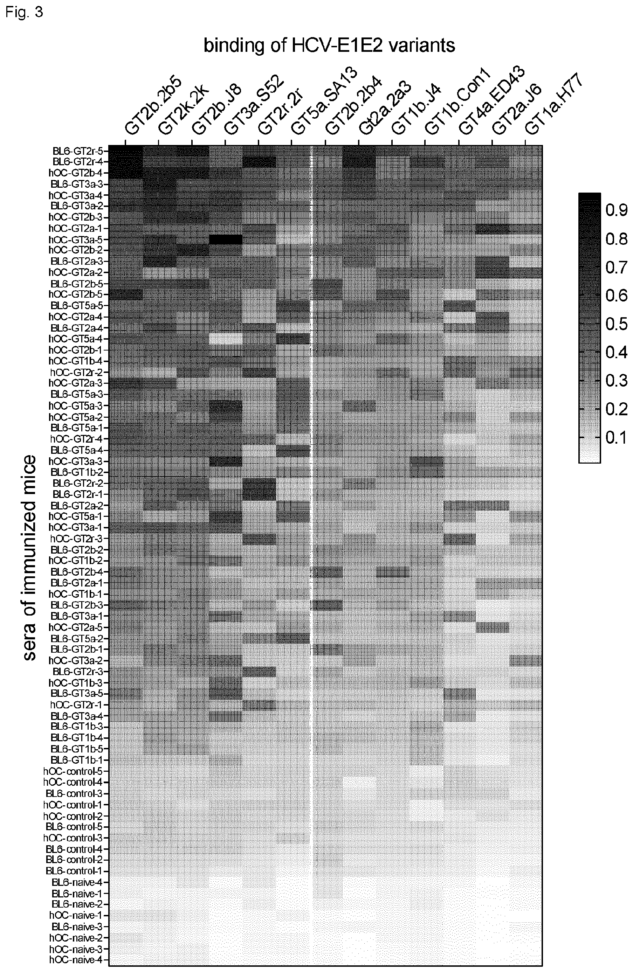

**Fig. 3****: ELISA-binding data of rVSV-HCV immunized mice sera to lysates of 13 HCV reporter virus infected cells representing the six established biotype clusters.** Ranking of the binding data of all 74 analyzed mouse serum samples (rows) against lysates of cells infected with 13 HCVcc reference viruses (columns) determined in the ELISA binding assay. The OD values are presented in a range between 0 and 1 (with 1 as the strongest binding in dark and 0 as the weakest binding in white).

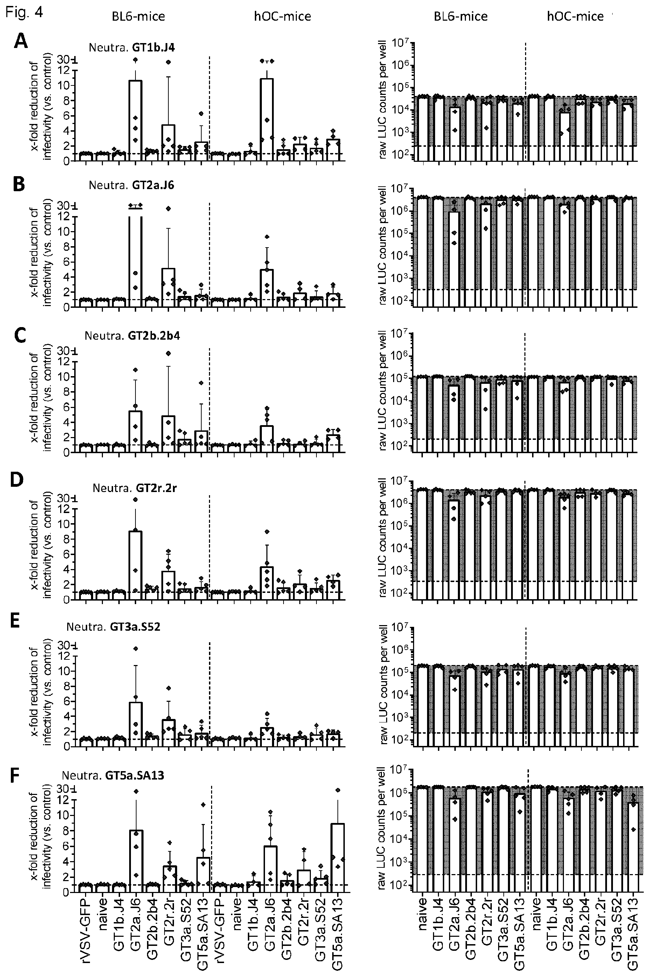

**Fig. 4****: Neutralization data of mouse sera vs. six representatives of the established biotype clustering and depiction of the dynamic assay range.** (A-F) Ability to neutralize the 6 HCV biotypes by a 1:20 dilution of all mouse sera from immunized BL6 and hOC animals. All data points represent measurement results of individual animals, bars the mean of a group and error bars the standard deviation (SD). The results are shown separately for BL6 mice and hOC mice. **Left side:** Representation of the neutralization of the reference viruses (one reference virus to be neutralized per figure) by mouse sera shown as x-fold neutralization compared to the mean value that was achieved by the sera of the control animals. **Right side:** Figure of the raw data of luciferase measurement as an indicator of the remaining infectivity of the reference viruses in a logarithmic representation. The dynamic range of this assay (light grey area) is defined by the mean remaining luciferase count after adding control mouse sera as the upper border (upper dotted line) and the background of the measurement system as the lower border (lower dotted line).

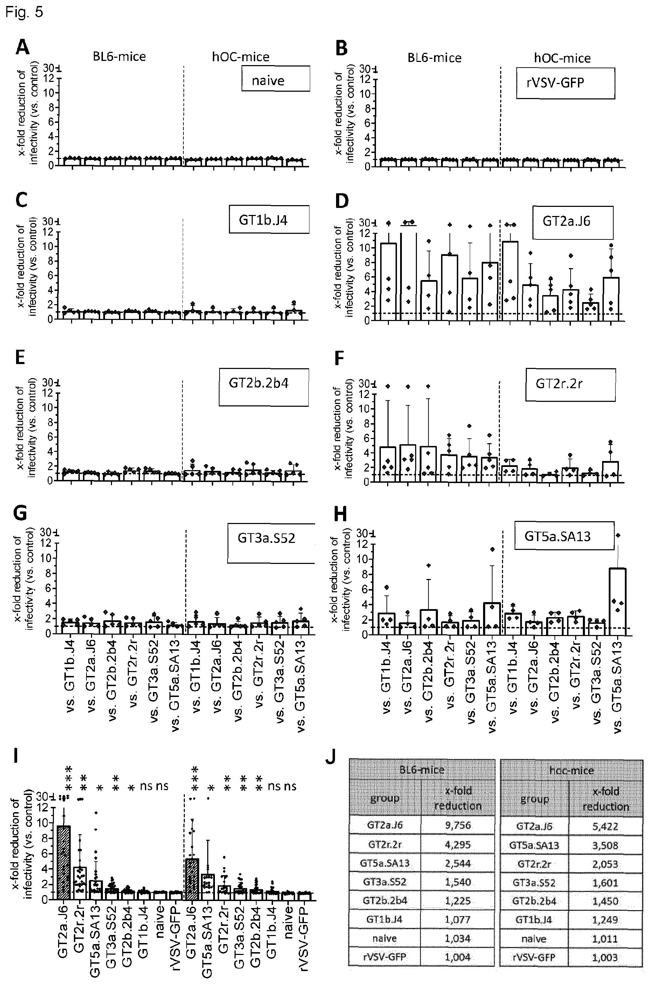

**Fig. 5****: Summary of all neutralization data and ranking of immunization groups.** (A-H) Neutralization indicated reference viruses by the mouse serum samples (one group of immunized animals per figure) as x-fold neutralization over the mean neutralization achieved by sera of the control animals. (I) Summary of data from (A-H). Neutralization data of all reference viruses for the different mouse groups are summarized and form the basis of a ranking of the candidates. Statistics calculated using the Brown-Forsythe and Welch ANOVA test (ns = not significant; *p<0.05; **p<0.01; ***p<0.001). For (A-I) the following applies: all data points represent measurement results of individual animals, bars the mean of a group and error bars the standard deviation (SD). The results are shown separately for BL6 mice and hOC mice. (J) Final ranking of the neutralization data of the individual immunized mouse groups shown as mean x-fold neutralization compared to the respective control group.

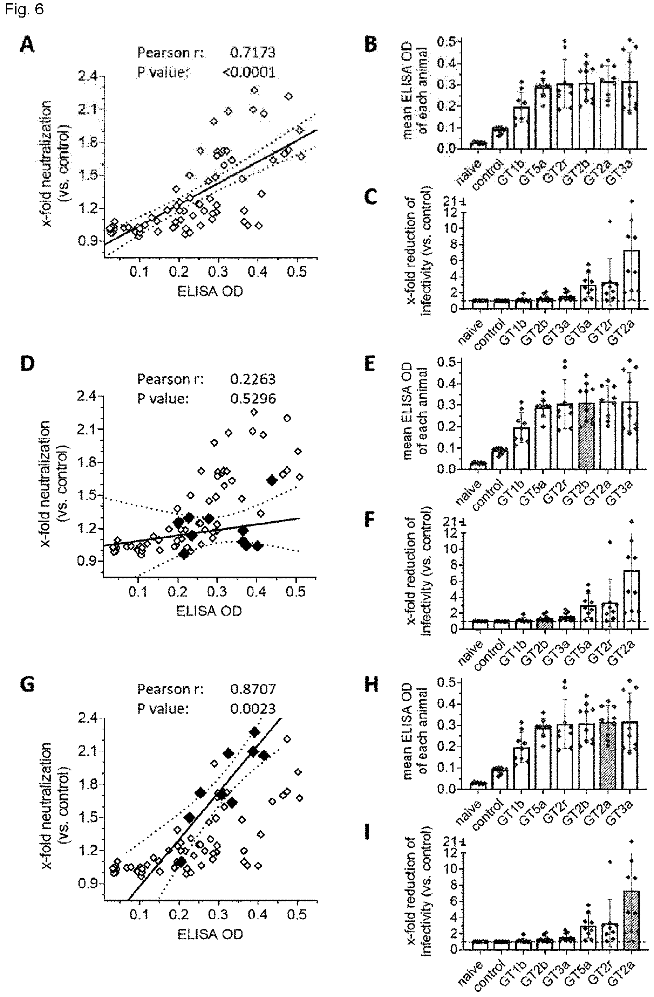

**Fig. 6****: Differential correlations of group specific binding capacity and neutralization capacity for of all tested representative reporter viruses.** (A) Correlation of the binding data (see figure 3) with the neutralization data (see figure 4 and figure 5) determined with sera of the immunized mice. Calculation of the correlation based on all 74 animals. The area between the dotted lines depicts the 95% confidence interval. (B) Summary of ELISA binding data. One data point represents the mean of the ELISA binding-assay OD against the lysates of cells infected with all 13 reference viruses tested. (C) Summary of neutralization data. One data point represents the mean neutralization of all 6 reference viruses tested. For (B-C), all data points represent measurements of individual animals, bars represent the mean of a group, and error bars represent the standard deviation. (D-F) see descriptions of (A-C). Difference: The correlation is only calculated using the n=10 animals of the GT2b.2b4-immunized group. (G-I) see descriptions of (A-C). Difference: The correlation is calculated only on the basis of the n=9 animals of the GT2a.J6-immunized group.
**Fig. 7****: Unique neutralization-fingerprints of rVSV-HCV-E1/E2 vaccine candidates using six monoclonal antibodies.** (A-C) Radar plots depicting the neutralization sensitivity of the HCVcc versions of the three best rVSV-HCV-constructs vs. six different monoclonal HCV-specific antibodies resulting in unique neutralization fingerprints. Values are plotted as percent neutralization (0% to 100%) of the specific HCVcc infection by the regarding monoclonal antibody used in a concentration of 50 µg/ml. Black line represents mean neutralization value of n=3 biological replicates and dotted lines are marking the plus and minus 10% interval.

### Sequences

| | |
|---|---|
| SEQ ID NO: 1 | HCV E1 protein of GT2a-J6 |
| SEQ ID NO: 2 | HCV E2 protein of GT2a-J6 |
| SEQ ID NO: 3 | 60 C-terminal aa of HCV core protein of GT2a-J6 |
| SEQ ID NO: 4 | HCV E1 protein of GT2r.2r |
| SEQ ID NO: 5 | HCV E2 protein of GT2r.2r |
| SEQ ID NO: 6 | 60 C-terminal aa of HCV core protein of GT2r.2r |
| SEQ ID NO: 7 | HCV E1 protein of GT5a.SA13 |
| SEQ ID NO: 8 | HCV E2 protein of GT5a.SA13 |
| SEQ ID NO: 9 | 60 C-terminal aa of HCV core protein of GT5a.SA13 |
| SEQ ID NO: 10 | nucleic acid encoding the 60 C-terminal amino acids of HCV core protein, E1 protein and E2 protein of GT2a-J6 |
| SEQ ID NO: 11 | nucleic acid encoding the 60 C-terminal amino acids of HCV core protein, E1 protein and E2 protein of GT2r.2r |
| SEQ ID NO: 12 | nucleic acid encoding the 60 C-terminal amino acids of HCV core protein, E1 protein and E2 protein of GT5a.SA13 |
| SEQ ID NO: 13 | rVSV based vector construct encoding the proteins of the VSV particles of the invention, based on GT2a-J6 |
| SEQ ID NO: 14 | rVSV based vector construct encoding the proteins of the VSV particles of the invention, based on GT2r.2r |
| SEQ ID NO: 15 | rVSV based vector construct encoding the proteins of the VSV particles of the invention, based on GT5a.SA13 |
| SEQ ID NO: 16 | VSV-G |

### Examples

### 1. Propagation and isolation/concentration of rVSV-HCV-E1/E2 particles

For propagation of rVSV-HCV-E1/E2 particles, transgenic BHK-G43 cells were seeded 24 h prior to infection. Mifepristone-dependent expression of VSV-G surface protein in BHK-G43 cells was induced 6 h prior to infection. For induction DMEM medium containing 5 % FCS and 10⁻⁹ M mifepristone was added and cells were incubated for 6 hours at 37°C. The rVSV-HCV-E1/E2 virus stock was added to the medium at a final dilution of 1:100 to 1:200. Supernatant was harvested after 24 hours and cell debris removed by low speed centrifuge. In order to remove the exhausted mifepristone containing cell culture medium, the particle containing supernatant was applied to a spin column-based purification system (Amicon^{®} Pro Purification System with 100kDa Amicon^{®} Ultra-0.5 Device). rVSV pseudo particles restrained by the filter unit were eluted with plain DMEM without additives or with PBS. Aliquots were prepared and frozen in liquid nitrogen. Titration was performed on BHK-21 cells (the basic cell line of the transgenic BHK-G43 cells).

### 2. Production of infectious cell culture derived Hepatitis C Virus stocks (HCVcc-stocks)

Up to 20 µg of reporter virus plasmid DNA was linearized using an appropriate restriction enzyme. Plasmid DNA was purified using the Qiagen Spin Miniprep kit according to the vendors' instructions. Subsequently, 2µg of restricted and purified plasmid DNA were used as template for *in vitro* transcription. Reactions were completed in a total volume of 100 µL containing the following components: 80 mM HEPES (pH 7.5), 12 mM MgCl₂, 2 mM spermidine, 40 mM dithiothreitol (DTT), a 3.125 mM concentration of each ribonucleoside triphosphate, 1 U RNase inhibitor (Promega), 0.6 U of T7 RNA polymerase (Promega) per µL. After incubation of the reaction mix for 2h at 37°C, 0.3 U T7 polymerase/µL was added and the reaction continued for2 additional hours at 37°C. Subsequently, transcription was terminated by addition of 7.5 U DNAse (Promega) and incubation for 30 minutes at 37°C. *In vitro* transcribed RNA was purified by using the NucleoSpin RNA Clean up kit (Macherey & Nagel) according to manufacturer's instructions. To generate HCVcc reporter virus particles, we electroporated Huh7.5.1 cells with 5µg of *in vitro* transcribed reporter virus RNA. Briefly, single-cell suspensions were prepared by trypsin-treatment and cells were washed with phosphate-buffered saline (PBS), counted and resuspended at a cell density of 10⁷ cells per mL in Cytomix. Cytomix is composed of 2mM ATP, 5mM glutathione, 120 mM KCI , 0.15 mM CaCl₂, 10 mM K₂HPO₄/KH₂PO₄ (pH 7.6); 25 mM Hepes, 2 mM EGTA, 5 mM MgCl₂. ATP and glutathione were added just prior to use. 400µL of the cell suspension in Cytomix were gently mixed with 5 µg in vitro transcribed RNA, transferred to an electroporation cuvette (gap width 0.4 cm; BioRad), and electroporated with a BioRad Gene-pulser using 975 µF, and 270 V settings. Electroporated cells were quickly transferred into fresh DMEM and seeded in cell culture vessels. We collected and pooled supernatants of electroporated cells 48, 72 and 96 h after electroporation, filtered them through a 0.45 µm filter and stored them at - 80°C.

### 3. HCVcc neutralization assay using HCV-specific monoclonal antibodies or mouse serum

For neutralization of an HCV infection, 100 µL of a Huh-7.5 cell suspension (10⁵ cells per ml) were seeded into a 96-well plate 24 h prior to inoculation. HCVcc reporter viruses were mixed with 50 µg/mL of monoclonal antibodies or with a 1:20 dilution of heat inactivated serum (30 min at 56°C) of immunized mice and incubated for at 37°C for 45 min. This mixture was afterwards used to inoculate cells for 4 h in triplicates at 37°C. Thereafter, 170 µL DMEM was added onto the cells. Infection was quantified 72 h after virus inoculation by measuring luciferase activity. To this end, cells were washed once with PBS and lysed directly on the plate by addition of 35 µL Milli Q water. After one freeze and thaw cycle, lysates were resuspended and after addition of luciferase substrate (1 µM colenterazine in water) relative light units (RLUs) were measured in a plate luminometer (Lumat LB Centro, Berhold, Germany). The neutralization data was analyzed using GraphPad Prism V9.0.0 (GraphPad Software, La Jolla, California, USA).

### 4. ELISA assay for detection of HCV-binding antibodies

To evaluate the antibody titers of sera from vaccinated mice, nunc-Immuno plates (Thermo Scientific) were coated with cell-lysates of HCV-infected cells. Briefly, Huh-7.5.1 cells were transfected with HCV viral RNA and cell lysates were harvested 96 hours post infection with RIPA buffer. Plates were pretreated with the Galanthus nivalis (GNA) lectin (500 ng/well). Wells were washed twice with TBST (20 mM Tris-HCI pH 7.5, 150 mM NaCl, 0.1% Tween-20, Sigma) and blocked with 100 µl/well of BLOTTO (5% non-fat dry milk, 5% normal goat serum in TBST buffer) and left for 1 h at room temperature. After subsequent washing with TBST, 50 µl of cellular lysate (1:10 dilution in BLOTTO) was used to coat each well of the plate. The mice anti-sera and AP33 monoclonal antibody were diluted in Blotto 1:200. The reaction was developed with the addition of anti-mouse IgG-HRP antibody (sigma 1:1000) and TMB substrate (Sigma) and quenched with 1 M sulfuric acid; absorbance measured at 450 nm (and 630 nm for unspecific background) with an ELISA plate reader (BioTech).

### 5. Comparison and analysis of antibodies induced by different rVSV-HCV-E1/E2 particles

For each of the six biotypes identified in Bankwitz et al, 2021, rVSV-HCV-E1/E2 pseudovirus particles encoding the functional E1 and E2 envelope proteins of the biotypes were generated. VSV-G was deleted from the VSV genome and replaced either by the sequence of GFP (control vector) or by the sequence of the C-terminal 60 amino acids of the HCV core protein plus HCV glycoprotein E1 plus HCV glycoprotein E2, as shown exemplarily in Fig. 1b. Six constructs were produced: genotype 1b isolate J4 (representing biotype cluster 4), genotype 2a isolate J6 (representing biotype cluster 6, cf. SEQ ID NO: 13)), genotype 2b isolate 2b4 (representing biotype cluster 3), genotype 2r isolate 2r (representing biotype cluster 2, cf. SEQ ID NO: 14), genotype 3a isolate S52 (representing biotype cluster 1) and genotype 5a isolate SA13 (representing biotype cluster 5, cf. SEQ ID NO: 15).

The particles were produced (as explained in Example 1). As the cells in which they are produced expresses VSV-G, the particles also comprise said glycoproein in addition to the HCV envelope proteins. Consequently, the particles can also infect cells via VSV-G. After infection, newly formed pseudovirus particles do not any more comprise VSV-G, as it is not encoded in the nucleic acid. Consequently, a second round of infection is only possible in cells expressing at least the minimal set of the human HCV entry receptors CD81 and occludin. Therefore, in *in vivo* immunization studies, receptor-transgenic hOC mice with liver-specific expression of the human HCV entry receptors CD81 and occludin were used in addition to C57BL/6 wild-type mice (in which a second round of infection is excluded).

The particles were titrated, and mice were infected with the particles according to the scheme shown in Fig. 2. The animals were immunized with 1×10⁸ rVSV-HCV pseudovirus particles per mL injection volume intramuscularly at the beginning of the experiment, after 4 weeks and after 7 weeks with an injected volume of 60 µL. Minimal amounts of blood were drawn at week 5 and week 6 of the experiment, and at week 9 the final maximal blood volume of each animal was drawn. The serum was isolated by centrifugation and used for subsequent assays. The presence of binding antibodies in the serum was tested using an ELISA binding assay against lysates of cells infected with HCVcc 13 reference viruses (as described in Example 4), the presence of neutralizing antibodies was tested using a neutralization assay against 6 different cell culture-generated reference viruses (as described in Example 3).

The results, shown in Fig. 3-6, show that all vectors were capable of inducing antibodies in the mice that can bind at least some of the diverse envelope protein systems of the test system, but there are fundamental differences with regard to the quality of these antibodies in particular, with regard to the induction of neutralising antibodies.

The sera of all 56 immunised mice show generation of HCV E1E2 binding antibodies, as shown in Fig. 2B. Reactivity of the sera of all immunised animals against 13 E1E2 envelope proteins of reference viruses previously characterized as belonging to different neutralisaiton clusters (Bankwitz et al., 2021) was quantitatively compared by an ELISA test (Fig. 3). A positive binding (defined as at least double OD compared to the control group) was found against at least one E1E2 complex of the 13 representatives of the 6 biotypes. In total, 28 of the 56 sera comprised antibodies capable of binding 12 or even all 13 of the used lysates, which proves the presence of very broadly binding antibodies.

As a central approach to further analysis of the estimated antiviral effect of these antibodies, the inventors used a neutralisation assay to demonstrate the presence of neutralising antibodies in the sera, as described above in Example 3. The neutralisation assay was analysed both with regard to internal quality and comparability (Fig. 4) and with regard to the capability of detecting neutralising antibodies (Fig. 5). Neutralisation was tested for the same 6 biotypes used to prepare the rVSV-HCV constructs for immunisation. All biotypes could successfully be neutralised by the corresponding mouse sera from both immunised mouse strains (Fig. 4, left). Additionally, the specific measuring window for neutralisation of each HCVcc was defined individually as dynamic range between the plate background and the level of neutralization of control sera (Fig. 4, right column, It was shown that the smallest window of analysis. For GT1b.J4, Fig. 4A can detect a reduction of infectivity up to a factor of 166-fold compared to the control, and the biggest window of analysis (GT2a.J6, Fig. 4B) a reduction of infectivity up to 13.043-fold compared to the control.

Fig. 5A-H describes the measured neutralisation of each group of imunised animals against all virus particles generated in cell culture. Fig. I-J summarise the results of each group of animals against all tested viruses. The assays did not show any neutralising activity (above background) of sera from naïve mice or from animals immunised with a GFP-expressing vector as a control (Fig. 5A-B). In the groups of immunisation with the VSV-constructs having envelope proteins of the genotypes GT1b.J4, GT2b.2b4 or GT3a.S52, only in some animals, a low level of neutralisation could be observed (Fig. 5C, E, G). In contrast, in animals immunized with GT2r.2r, GT5a.SA13 and GT2a.J6 (Fig. 5D, F and H), a clear neutralisation by the sera was detected.

The sera from the group of animals immunised with GT2a.J6 are the most promising group (Fig. 5D).As the average of all tested viruses, in BI6 mice, this group led to a 9.8-fold higher and highly significant reduction in infectivity compared to the sera of control mice. In hOC mice, a 5.4-fold higher and also highly significant reduction was observed (Fig. 5I-J). This rVSV-HCV-GT2a.J6 vaccination vector induced highly efficient neutralising antibodies against all 6 reference viruses representing all 6 different neutralisation clusters/biotypes of HCV (Fig. 5D).

An additional important parameter is the correlation of the binding characteristics with the neutralisation characteristics of the antibodies induced by immunisation with the candidate vaccination vectors (Fig. 6). On the basis of all animals (n=74), there is a highly significant (P<0.0001) positive correlation between the measured binding and the measured neutralisaiton of the mouse sera (r=0.7173), Fig. 6A. Between the vaccine candidates, there are however clear differences: With rVSV-HCV-E1E2-GT2b.2b4 immunised animals, only a low correlation is seen (r=0.2263). With increasing binding capabilities of the generated antibodies, the strong binding characteristics (Fig. 6E, marked) could not be transformed into strong neutralisation characteristics (Fig. 6F, marked). In contrast, the animals immunised with rVSV-HCV-GT2a.J6 show the stongest positive correlation between groups (r=0.8707, Fig. 6G, marked). The good binding characteristics of the antibodies (Fig. 6H, marked) correspond to the stringes neutralisation characteristics of the whole analysis (Fig. 6I, marked). Thus, both vectors discussed here induce antibodies that can bind to different E1E2 variants. However, rVSV-HCV-GT2a.J6 is particularly effective with regard to the induction of neutralising antibodies capable of neutralising HCV variants from different biotypes.

This confirms that rVSV-HCV expressing E1 and E2 proteins from a HCV strain from GT2a.J6 (cf. Fig. 1A)), e.g., the rVSV-HCV-GT2a.J6, is most suitable as a vaccine candidate.

## Claims

1. rVSV-HCV pseudovirus particles comprising
a) at least the 60 C-terminal amino acids of HCV core protein,
b) HCV E1 protein of a first HCV strain
c) HCV E2 protein of a first HCV strain, and
d) VSV-G protein,
wherein the first HCV strain is a HCV strain selected from GT2a.J6, GT2r.2r and GT5a.SA13.

2. The particles of claim 1, wherein HCV is HCV strain GT2a.J6.

3. The particles of claim 2, wherein the HCV E1 protein has at least 90% amino acid identity to SEQ ID NO: 1, and/or the HCV E2 protein has at least 90% amino acid identity to SEQ ID NO: 2, optionally, excluding aa 1-27,
wherein, preferably, the sequence of the HCV E1 protein is SEQ ID NO: 1, and/or the sequence of the HCV E2 protein is SEQ ID NO: 2, optionally, excluding aa 1-27.

4. The particles of any of the preceding claims, wherein the HCV core protein also is a HCV core protein of said first HCV strain,
wherein, preferably, the sequence of the 60 C-terminal amino acids of the HCV core protein is SEQ ID NO: 3, and wherein the sequence of the HCV E1 protein is SEQ ID NO: 1, and the sequence of the HCV E2 protein is SEQ ID NO: 2, optionally, excluding aa 1-27.

5. The particles of any of the preceding claims, further comprising VSV nucleoprotein, VSV phosphoprotein, VSV matrixprotein, and VSV polymerase.

6. The particles of any of the preceding claims, further comprising a nucleic acid encoding said 60 C-terminal amino acids of HCV core protein, HCV E1 protein and HCV E2 protein, VSV nucleoprotein, VSV phosphoprotein, VSV matrixprotein, and VSV polymerase.

7. The particles of any of the preceding claims, not comprising a nucleic acid encoding VSV-G protein.

8. The particles of any of the preceding claims, comprising at least one further HCV protein selected from the group consisting of P7, N2, NS3, NS4A, NS4B, NS5A and NS5B.

9. A composition comprising particles of any of the preceding claims, and, optionally, a suitable solvent and/or excipient.

10. A composition particles of claim 9, optionally, further comprising particles comprising
a) at least the 60 C-terminal amino acids of HCV core protein,
b) HCV E1 protein of a second HCV strain and
c) HCV E2 protein of a second HCV strain,
d) VSV-G protein,
wherein said second HCV strain is different from said first HCV strain,
wherein, optionally, said second HCV strain is a HCV strain selected from GT2a.J6, GT2r.2r and GT5a.SA13.

11. A nucleic acid encoding
a) at least the 60 C-terminal amino acids of HCV core protein,
b) HCV E1 protein of a first HCV strain,
c) HCV E2 protein of a first HCV strain, and
d) VSV nucleoprotein, VSV phosphoprotein, VSV matrixprotein, and VSV polymerase,
wherein the first HCV strain is a HCV strain selected from GT2a.J6, GT2r.2r and GT5a.SA13.

12. A method for producing rVSV-HCV pseudovirus particles, comprising
a) culturing a cell expressing VSV-G protein and transfected with the nucleic acid of claim 11 under conditions suitable for production of said particles, and
b) isolating said particles, and
c) concentrating said particles, and
d) optionally, formulating said particles in a formulation suitable for storage and/or administration as a vaccine.

13. rVSV-HCV particles obtainable by the method of claim 12.

14. A vaccine comprising the rVSV-HCV particles of any of claims 1-8 or 13, the nucleic acid of claim 11, or the composition of any of claims 9-10.

15. The vaccine of claim 14 for use in prophylactic vaccination of a subject, optionally, a subject that has successfully been treated for a chronic HCV infection.

## Patentansprüche

1. rVSV-HCV-Pseudoviruspartikel, umfassend
a) mindestens die 60 C-terminalen Aminosäuren von HCV-Kernprotein,
b) HCV-E1-Protein eines ersten HCV-Stamms
c) HCV-E2-Protein eines ersten HCV-Stamms und
d) VSV-G-Protein,
wobei der erste HCV-Stamm ein HCV-Stamm ist, der aus GT2a.J6, GT2r.2r und GT5a.SA13 ausgewählt ist.

2. Die Partikel nach Anspruch 1, wobei HCV der HCV-Stamm GT2a.J6 ist.

3. Die Partikel nach Anspruch 2, wobei das HCV-E1-Protein mindestens 90 % Aminosäureidentität zu SEQ ID NO: 1 und/oder das HCV-E2-Protein mindestens 90 % Aminosäureidentität zu SEQ ID NO: 2 hat, optional unter Ausschluss von AS 1-27,
wobei vorzugsweise die Sequenz des HCV-E1-Proteins SEQ ID NO: 1 ist und/oder die Sequenz des HCV-E2-Proteins SEQ ID NO: 2 ist, optional unter Ausschluss von AS 1-27.

4. Die Partikel nach einem der vorstehenden Ansprüche, wobei das HCV-Kernprotein auch ein HCV-Kernprotein des besagten ersten HCV-Stamms ist,
wobei vorzugsweise die Sequenz der 60 C-terminalen Aminosäuren des HCV-Kernproteins SEQ ID NO: 3 ist und wobei die Sequenz des HCV-E1-Proteins SEQ ID NO: 1 ist und die Sequenz des HCV-E2-Proteins SEQ ID NO: 2 ist, optional unter Ausschluss von AS 1-27.

5. Die Partikel nach einem der vorstehenden Ansprüche, die zusätzlich VSV-Nukleoprotein, VSV-Phosphoprotein, VSV-Matrixprotein und VSV-Polymerase umfassen.

6. Die Partikel nach einem der vorstehenden Ansprüche, die ferner eine Nukleinsäure umfassen, kodierend für die besagten 60 C-terminalen Aminosäuren des HCV-Kernproteins, HCV-E1-Protein und HCV-E2-Protein, VSV-Nukleoprotein, VSV-Phosphoprotein, VSV-Matrixprotein und VSV-Polymerase.

7. Die Partikel nach einem der vorstehenden Ansprüche, keine Nukleinsäure umfassend, die VSV-G-Protein kodiert.

8. Die Partikel nach einem der vorstehenden Ansprüche, umfassend mindestens ein weiteres HCV-Protein, ausgewählt aus der Gruppe bestehend aus P7, N2, NS3, NS4A, NS4B, NS5A und NS5B.

9. Eine Zusammensetzung, die Partikel nach einem der vorstehenden Ansprüche und optional ein geeignetes Lösungsmittel und/oder Hilfsstoff umfasst.

10. Eine Zusammensetzung von Partikeln nach Anspruch 9, die optional zusätzlich Partikel umfasst, umfassend
a) mindestens die 60 C-terminalen Aminosäuren von HCV-Kernprotein,
b) HCV-E1-Protein eines zweiten HCV-Stamms und
c) HCV-E2-Protein eines zweiten HCV-Stammes,
d) VSV-G-Protein,
wobei sich der besagte zweite HCV-Stamm vom besagten ersten HCV-Stamm unterscheidet,
wobei der besagte zweite HCV-Stamm optional ein HCV-Stamm ist, der aus GT2a.J6, GT2r.2r und GT5a.SA13 ausgewählt ist.

11. Eine Nukleinsäure, kodierend für
a) mindestens die 60 C-terminalen Aminosäuren von HCV-Kernprotein,
b) HCV-E1-Protein eines ersten HCV-Stammes,
c) HCV-E2-Protein eines ersten HCV-Stammes und
d) VSV-Nukleoprotein, VSV-Phosphoprotein, VSV-Matrixprotein und VSV-Polymerase,
wobei der erste HCV-Stamm ein HCV-Stamm ist, der aus GT2a.J6, GT2r.2r und GT5a.SA13 ausgewählt ist.

12. Ein Verfahren zur Herstellung von rVSV-HCV-Pseudoviruspartikeln, umfassend
a) Kultivierung einer Zelle, die VSV-G-Protein exprimiert und mit der Nukleinsäure nach Anspruch 11 transfiziert ist, unter zur Herstellung der besagten Partikel geeigneten Bedingungen,
b) Isolierung besagter Partikel und
c) Konzentration besagter Partikel und
d) optional, Formulierung besagter Partikel in einer Formulierung, die zur Lagerung und/oder Verabreichung als Impfstoff geeignet ist.

13. rVSV-HCV-Partikel, die durch das Verfahren nach Anspruch 12 erhältlich sind.

14. Ein Impfstoff, der die rVSV-HCV-Partikel nach einem der Ansprüche 1-8 oder 13, die Nukleinsäure nach Anspruch 11 oder die Zusammensetzung nach einem der Ansprüche 9-10 umfasst.

15. Der Impfstoff nach Anspruch 14 zur Verwendung bei der prophylaktischen Impfung eines Subjekts, optional eines Subjekts, das erfolgreich wegen einer chronischen HCV-Infektion behandelt wurde.

## Revendications

1. Particules de pseudovirus rVSV-VHC comprenant
a) au moins les 60 acides aminés d'extrémité C-terminale de la protéine Core du VHC,
b) la protéine E1 de VHC d'une première souche de VHC
c) la protéine E2 de VHC d'une première souche de VHC, et
d) la protéine VSV-G,
dans lesquelles la première souche de VHC est une souche de VHC choisie parmi GT2a.J6, GT2r.2r et GT5a.SA13.

2. Particules selon la revendication 1, dans lesquelles le VHC est la souche de VHC GT2a.J6.

3. Particules selon la revendication 2, dans lesquelles la protéine E1 de VHC a au moins 90 % d'identité d'acides aminés vis-à-vis de la SEQ ID NO : 1, et/ou la protéine E2 de VHC a au moins 90 % d'identité d'acides aminés vis-à-vis de la SEQ ID NO : 2, éventuellement, à l'exclusion des aa 1 à 27,
dans lesquelles, de préférence, la séquence de la protéine E1 de VHC est la SEQ ID NO : 1, et/ou la séquence de la protéine E2 de VHC est la SEQ ID NO : 2, éventuellement, à l'exclusion des aa 1 à 27.

4. Particules selon l'une quelconque des revendications précédentes, dans lesquelles la protéine Core de VHC est également une protéine Core de VHC de ladite première souche de VHC,
dans lesquelles, de préférence, la séquence des 60 acides aminés d'extrémité C-terminale de la protéine Core de VHC est la SEQ ID NO : 3, et dans lesquelles la séquence de la protéine E1 de VHC est la SEQ ID NO : 1, et la séquence de la protéine E2 de VHC est la SEQ ID NO : 2, éventuellement, à l'exclusion des aa 1 à 27.

5. Particules selon l'une quelconque des revendications précédentes, comprenant en outre la nucléoprotéine de VSV, la phosphoprotéine de VSV, la protéine de matrice de VSV et la polymérase de VSV.

6. Particules selon l'une quelconque des revendications précédentes, comprenant en outre un acide nucléique codant pour lesdits 60 acides aminés d'extrémité C-terminale de la protéine Core de VHC, la protéine E1 de VHC et la protéine E2 de VHC, la nucléoprotéine de VSV, la phosphoprotéine de VSV, la protéine de matrice de VSV et la polymérase de VSV.

7. Particules selon l'une quelconque des revendications précédentes, ne comprenant pas un acide nucléique codant pour la protéine VSV-G.

8. Particules selon l'une quelconque des revendications précédentes, comprenant au moins une protéine de VHC supplémentaire choisie dans le groupe constitué par P7, N2, NS3, NS4A, NS4B, NS5A et NS5B.

9. Composition comprenant des particules selon l'une quelconque des revendications précédentes, et, éventuellement, un solvant et/ou excipient adapté.

10. Composition des particules selon la revendication 9, éventuellement comprenant en outre des particules comprenant
a) au moins les 60 acides aminés d'extrémité C-terminale de la protéine Core du VHC,
b) la protéine E1 de VHC d'une seconde souche de VHC et
c) la protéine E2 de VHC d'une seconde souche de VHC,
d) la protéine VSV-G,
dans lesquelles ladite seconde souche de VHC est différente de ladite première souche de VHC,
dans lesquelles, éventuellement ladite seconde souche de VHC est une souche de VHC choisie parmi GT2a.J6, GT2r.2r et GT5a.SA13.

11. Acide nucléique codant pour
a) au moins les 60 acides aminés d'extrémité C-terminale de la protéine Core du VHC,
b) la protéine E1 de VHC d'une première souche de VHC,
c) la protéine E2 de VHC d'une première souche de VHC, et
d) la nucléoprotéine de VSV, la phosphoprotéine de VSV, la protéine de matrice de VSV et la polymérase de VSV,
dans lequel la première souche de VHC est une souche de VHC choisie parmi GT2a.J6, GT2r.2r et GT5a.SA13.

12. Procédé de production de particules de pseudovirus rVSV-VHC, comprenant les étapes consistant à
a) mettre en culture une cellule exprimant la protéine VSV-G et transfectée par l'acide nucléique selon la revendication 11 dans des conditions adaptées à la production desdites particules, et
b) isoler lesdites particules, et
c) concentrer lesdites particules, et
d) éventuellement, formuler lesdites particules en une formulation adaptée au stockage et/ou à l'administration en tant que vaccin.

13. Particules de rVSV-VHC pouvant être obtenues par le procédé selon la revendication 12.

14. Vaccin comprenant les particules de rVSV-VHC selon l'une quelconque des revendications 1 à 8 ou 13, l'acide nucléique selon la revendication 11, ou la composition selon l'une quelconque des revendications 9 à 10.

15. Vaccin selon la revendication 14 pour une utilisation dans la vaccination prophylactique d'un sujet,
éventuellement, un sujet qui a été traité avec succès pour une infection chronique par le VHC.
